Europäisches Patentamt

European Patent Office

Office européen des brevets

(11) Veröffentlichungsnummer : **0 542 786 B1**

# (12) EUROPÄISCHE PATENTSCHRIFT

(45) Veröffentlichungstag der Patentschrift :
**01.06.94 Patentblatt 94/22**

(21) Anmeldenummer : **91913661.4**

(22) Anmeldetag : **26.07.91**

(86) Internationale Anmeldenummer :
**PCT/EP91/01402**

(87) Internationale Veröffentlichungsnummer :
**WO 92/02204 20.02.92 Gazette 92/05**

(51) Int. Cl.⁵ : **A61K 7/04**, B32B 27/08

(54) **SELBSTKLEBENDES LAMINAT FÜR NÄGEL.**

(30) Priorität : **30.07.90 DE 4024125**

(43) Veröffentlichungstag der Anmeldung :
**26.05.93 Patentblatt 93/21**

(45) Bekanntmachung des Hinweises auf die
Patenterteilung :
**01.06.94 Patentblatt 94/22**

(84) Benannte Vertragsstaaten :
**AT BE CH DE DK ES FR GB GR IT LI LU NL SE**

(56) Entgegenhaltungen :
**DE-A- 3 337 458**

(73) Patentinhaber : **LTS LOHMANN
THERAPIE-SYSTEME GmbH & CO.KG
Postfach 23 43
D-56513 Neuwied (DE)**

(72) Erfinder : **HOFFMANN, Hans-Rainer
Burghofstra e 123
D-5450 Neuwied 22 (DE)**
Erfinder : **VON KLEINSORGEN, Reinhard
Flurstra e 45a
D-5450 Neuwied 2 (DE)**
Erfinder : **SIMON, Günter
Tulpenweg 1
D-5533 Hillesheim (DE)**
Erfinder : **STEINBORN, Dorothea
Weingartenstra e 33a
D-5450 Neuwied 22 (DE)**

(74) Vertreter : **Flaccus, Rolf-Dieter, Dr.
Patentanwalt
Sperlingsweg 32
D-50389 Wesseling (DE)**

EP 0 542 786 B1

**EP 0 542 786 B1**

**Beschreibung**

Die Erfindung betrifft ein selbstklebendes, wenigstens einen Weichmacher enthaltendes, an Fuß- und/oder Fingernägel anformbares Laminat, enthaltend

a) eine Farbstoffe und/oder Pigmente enthaltende filmbildende Polymerschicht

b) eine darauf befindliche Haftkleberschicht und

c) eine die Haftkleberschicht abdeckende, wiederablöbare, vorzugsweise silikonisierte Schutzfolie,

ein Verfahren zu seiner Herstellung sowie seine Verwendung zur Anbringung auf Fuß- bzw. Fingernägeln als künstliche Nägel.

Es ist bekannt, Nägel mittels lösungsmittelhaltigen Polymerlacken zur Erzielung eines kosmetischen Effektes zu behandeln (Nagellack). Ebenfalls ist bekannt, einem solchen Lack einen antimykotisch wirksamen Stoff bzw. einen den Nagel pflegenden Wirkstoff zuzusetzen.

Auch ist es allgemein bekannt, wieviel Mühe es bereitet, einen solchen Nagellack mit dem gewünschten Erscheinungsbild aufzutragen. (Zeit, Trocknung des Lacks, Übung, äußeres Erscheinungsbild).

Der Gehalt der bekannten Nagellacke an organischen Lösungsmitteln stellt angesichts des hohen weltweiten Verbrauchs eine erhebliche Umweltbelastung dar, die aus Gründen des Umweltschutzes vermieden oder wenigstens verringert werden sollte. Allein schon aus diesem Grund sollte der Einsatz einer dekorativen Folie zur Applikation auf Nägel, verbunden mit den vom Verbraucher geforderten Eigenschaften wie Glanzeffekt, Farbeffekt, Haltbarkeit, Tragbarkeit und Natürlichkeit als Ersatz für den lösungsmittelhaltigen Lack als sinnvolle Alternative anerkannt werden.

Der Einsatz künstlicher Nägel, wie auch immer mit dem Nagel verbunden, ist bekannt.

Aus der DE-OS 23 62 818 ist es auch bekannt, Nägel mit einer haftenden Folie aus PVC zu bekleben. Ein derartiges System hat jedoch im Markt keine Akzeptanz gefunden, weil PVC als Polymermaterial nicht die Verbrauchererwartungen hinsichtlich Glanz, Härte, Flexibilität und Haltbarkeit erfüllt. Die hohe Reißfestigkeit der PVC-Folie oder ähnlicher Polymerfilme wie Polyethylen, Polypropylen oder Polyester erschweren eine Anformbarkeit der Foie auf den Nagel durch einfaches Abscheren und bergen die Gefahr der Verletzung des Nagelbettes.

Die Anformbarkeit auf dem Nagel wie auch die Stabilität im Hinblick auf die Applikationsfähigkeit stehen in einem direkten Zusammenhang mit der Sprödigkeit des Laminats, wobei festgestellt werden mußte, daß die idealen Eigenschaften eines Nagellacklaminats (Laminat auf Basis Nitrocellulose - käuflicher Nagellack) Stabilitätsprobleme aufwirft, derart, daß das Laminat bei Lagerung brüchig wird, austrocknet und dann nicht mehr zu applizieren ist.

Aus der DE-A 33 37 458 ist eine selbstklebende, vorgefertigte Nageldekorschicht oder -folie für die dekorative Nagelkosmetik bekannt. Es handelt sich dabei um ein selbstklebendes Laminat mit einer Farbstoffe und/oder Pigmente enthaltenden, filmbildende Polymerschicht aus Grund- und Decklack, deren Klebstoffschicht mit einer abdeckenden, ablösbaren Schutzfolie bedeckt ist, und mit einer gegenüber den übrigen Laminatbestandteilen resistenten Abdeckfolie mit Trennmittel auf dem Decklack. Für die Applikation muß die Schutzfolie abgelöst werden. Hierbei können sich Schwierigkeiten ergeben, wenn das Adhäsionsvermögen der Schutzfolie etwa gleich dem Ahhäsionsvermögen der Abdeckfolie oder sogar noch größer ist.

Es ist Aufgabe der vorliegenden Erfindung, ein selbstklebendes, dem Nagel anformbares Laminat anzugeben, welches mit den im Handel befindlichen Nagellacken auf Basis von Lösungsmitteln vergleichbar ist, die vom Verbraucher gewünschten Eigenschaften wie Glanz, Oberflächenhärte, Flexibilität, Haltbarkeit auf dem Nagel ohne Fremdkörpergefühl aufweist und zusammen mit den vorgenannten Eigenschaften eine ausreichende Stabilität gegen Austrocknung und Brüchigkeit besitzt, sowie nach der Applikation ein problemloses Abziehen der Schutzfolie gewährleistet.

Die Lösung dieser Aufgabe, insbesondere die Vereinigung von hinreichender Stabilität bei gleichzeitig ausreichender Oberflächenhärte und Flexibilität erwies sich als schwierig, da diese Eigenschaften einander zuwider laufen. Erreicht man ein stabiles Laminat, so weist dieses aufgrund hohen Weichmacherzusatzes keine ausreichende Oberflächenhärte auf. Bei genügender Oberflächenhärte wird das Laminat dagegen bei Lagerung so schnell spröde, daß eine Applikation aufgrund Bruch des Laminats nicht mehr möglich war.

Überraschenderweise wurde gefunden, daß die Kombination dieser für die Anwendung wichtigen Eigenschaften erreicht wird, wenn man einen Lack-, bevorzugt einen Lack auf Nitrocellulose und/oder Acrylatbasis, zunächst auf eine Abdeckfolie aufbringt, sodann den Lack unter Rückgewinnung der Lösungsmittel trocknet und diese Kombination mit einem Kleberfilm und einer Schutzschicht laminiert.

Gegenstand der Erfindung ist somit ein selbstklebendes, wenigstens einen Weichmacher enthaltendes, an Fuß- und/oder Fingernägel anformbares Laminat, enthaltend

a) eine Farbstoffe und/oder Pigmente enthaltende filmbildende Polymerschicht

b) eine darauf befindliche Haftkleberschicht und

2

c) eine die Haftkleberschicht abdeckenden, wiederablösbaren, vorzugsweise silikonisierten Schutzfolie, dadurch gekennzeichnet, daß die filmbildende Polymerschicht auf der der Haftkleberschicht entgegengesetzten Seite mit einer abdeckenden, ablösbaren, gegenüber den übrigen Bestandteilen des Laminats und den bei der Herstellung des Laminats verwendeten Materialien resistenten Abdeckfolie versehen ist, wobei die vor Applikation zu entfernende Abdeckfolie auf einer beschichteten Seite adhäsiv ausgerüstet und die Adhäsion der Abdeckfolie an der Polymerschicht geringer ist, als die Adhäsion zwischen Kleberschicht und Schutzfolie.

Die Beschichtung des Lackes (d.h. der Polymerschicht) auf die vor der Applikation zu entfernende Abdeckschicht ermöglicht einerseits eine Strukturierung der Oberfläche der Polymerschicht und andererseits stellt sie die Stabilität und Gebrauchsfähigkeit des Laminats bis zur Applikation sicher. Durch den graduellen Unterschied der Adhäsion zwischen Abdeckfolie und Polymerschicht einerseits und Schutzfolie sowie Kleberschicht andererseits wird die Applikation des Laminats wesentlich erleichtert.

Um dieses problemlos zu erreichen, ist die Abdeckschicht auf der mit der Polymerschicht beschichteten Seite bevorzugt abhäsiv ausgerüstet, wodurch die Adhäsion zwischen Abdeckschicht und Laminat geringer ist als die Adhäsion zwischen Kleberschicht und Schutzfolie. Vorteilhaft wird die Abdeckschicht mit einer Abziehhilfe versehen, was die Handhabung erleichtert. Die Kleberschicht des Laminats kann aus einer bekannten Klebemasse bestehen. Das Flächengewicht der Kleberschicht liegt im Bereich von 20 bis 100 g/m$^2$, vorzugsweise bei 25 bis 75 g/m$^2$.

Die Polymerschicht des Laminats, die ein- oder ebenfalls mehrschichtig sein kann, weist eine Schichtdicke von 25 bis 200, vorzugsweise 60 bis 150 $\mu$m auf, wobei bei mehrschichtiger Ausbildung die einzelnen Schichten eine unterschiedliche Zusammensetzung aufweisen können.

Der Anteil des Weichmachers in der Polymerschicht (auch die Kleberschicht kann den gleichen oder einen anderen Weichmacher enthalten), beträgt 1 bis 30, bevorzugt 5 bis 12,5 und insbesondere 7 bis 10 Gew.-%, bezogen auf die Polymere und falls vorhanden, Pigmente des Lackes bzw. auf die Klebstoffmatrix.

Als Weichmacher werden bevorzugt Citronensäureester verwendet. Zusätzlich kann als Weichmacher Campher vorhanden sein. Der Zusatz von Campher bewirkt, daß die Folie nach Applikation durch Verdampfen des Camphers nachhärten kann. Der Verlust an Campher vor Applikation wird vermieden durch die Abdeckschicht.

Die Polymerschicht oder die Polymerschichten enthalten oder bestehen vorteilhaft aus Nitrocellulose allein oder Acrylaten allein oder Gemischen dieser beiden an sich für Nagellacke bekannten Komponenten.

Der Polymerschicht und/oder der Kleberschicht können Zusätze wie geruchsaktive Komponenten und/oder auch Wirkstoffe, vorzugsweise antimykotisch wirksame Stoffe wie Clotrimazol, Miconazol, Ketoconazol, Naftifin, Ciclopiroxolamin, Fenticlor, Sulbentin, Tolnaftat und Haloprogin zugesetzt werden.

Ferner kann die Kleberschicht eine Substanz enthalten, die die Penetration des Wirkstoffs in den Nagel fördert. Der Zusatz von Penetrationsbeschleunigern, -verstärkern (Enhancern) zu einer Wirkstoff/Kleber-Rezeptur ist aus dem Bereich der Transdermalen Therapeutischen Systeme bekannt. Substanzen, die einen Wirkstofftransport in die Haut bzw. durch die Haut fördern sind z.B. Dimethylsulfoxid (DMSO) und Dimethylformamid (DMF) wie auch eine große Anzahl verschiedener Emulgatoren, Fettsäuren und deren Ester.

Gegenstand der Erfindung ist ferner ein Verfahren zur Herstellung der selbstklebenden Laminate, das dadurch gekennzeichnet ist, daß man einen Lack, der Farbstoffe und/oder Pigmente, wenigstens einen Weichmacher und wenigstens ein filmbildendes Polymer, gelöst in einem organischen Lösungsmittel enthält, auf eine entfernbare Abdeckfolie aufbringt, die wenigstens die gleiche Größe wie das Laminat hat, den Lack unter Rückgewinnung des Lösungsmittels trocknet und die mit dem getrockneten Lack beschichtete Abdeckfolie lackseitig mit einem Kleberfilm und einer Schutzschicht laminiert.

Die Erfindung weist die folgenden Vorteile auf: Es wird kein organisches Lösungsmittel an die Umwelt abgegeben. Die erfindungsgemäßen, als künstliche Fuß- oder Fingernägel geeigneten Laminate erfüllen alle vom Verbraucher an sie gestellten Anforderungen hinsichtlich Glanz, Härte, Flexibilität und Haltbarkeit und vermeiden die Verletzung des Nagelbettes. Darüber hinaus kann die Polymerschicht durch die Verwendung einer geeignet strukturierten Abdeckschicht auch für dekorative Zwecke struktuiriert werden. Schließlich betrifft die Erfindung auch die Verwendung der erfindungsgemäßen Laminate als künstliche Fuß- oder Fingernägel.

Die Erfindung wird durch das nachfolgende Beispiel in nicht einschränkender Weise erläutert:

Beispiel:

1. Eine Klebemasse bestehend aus gleichen Anteilen an

Polyisobutylen

EP 0 542 786 B1

festes aliphatisches Kohlenwasserstoffharz
hydriertes Kolophoniumharz
0,01     Anteil Stabilisator (Antioxidans)
0,05     Anteil Weichmacher (Citronensäureester)
10       Anteile Spezialbenzin
wird auf eine einseitig abhäsiv ausgestattete PES-Folie aufgetragen und getrocknet (Kleberschichtdicke: 80 μm).

(Laminat 1)

    1. Klebmasse:
0,05 kg      Weichmacher
0,153 kg     Polyisobutylen (mittleres Molekulargewicht von 900.000 bis 1.400.000
0,137 kg     festes aliphatisches Kohlenwasserstoffharz
0,137 kg     hydriertes Kollophoniumharz
0,005 kg     Stabilisator (Antioxidans)
1,148 kg     Spezialbenzin 80-110 als Lösunsgmittel
    2. Eine im Handel erhältliche Nagellackmasse bestehend aus
4        Anteilen Cellulosenitrat
1        Anteil Acrylat-Copolymer
1        Anteil Sulfonamid-Harz
0,1      Anteil Campher
1        Anteil Weichmacher (Citronensäureester)
3        Anteile Farbpigment
30       Anteile Lösungsmittel
wird auf die Abdeckschicht (Polyesterfolie 50 μm) aufgetragen und getrocknet (Dicke der Polymerschicht 200 μm).

```
136,5 g          Nitrocellulose

 73,5 g          Butanol

 50,0 g          Toluolsulfonamidharz (Santolite)

 50,0 g          Polyacrylester (Acronal)

 30,0 g          Campher

 23,0 g          Weichmacher (Citronensäureester)

 60,0 g          Dibutylphthalat

 100 g           Butylacetat

 200 g           Ethylacetat

 200 g           Toluol

  77 g           Ethanol
────────
1000 g

  20 g           Farbpigmente
```

(Laminat 2)

Nitrocellulose: Filmbildner (Hart-Polymer)
Polyacrylester: Filmbildner (Weich-Polymer)
Sulfonamidharz: Glanzverstärker
    3. Laminat 1 und 2 werden anschließend derart aufeinander kaschiert, daß Kleberschicht und Polymerschicht verbunden sind.

(Laminat 3)

4. In eine silikonisierte und mit Aluminium bedampfte Polyesterfolie (Schutzschicht) werden halbrunde Einschnitte (Abziehhilfe) eingesetzt.

(Vollstanzung durch die Folie - Abb. 4)

5. Auf die Schutzschicht nach 4) wird das Laminat 3 kaschiert, wobei vorher vom Laminat 3 die PES-Folie des Laminats 1 entfernt wird, so daß auf die Schutzschicht die Kleberschicht zu liegen kommt.

6. In das so erhaltene Laminat werden von der Abdeckschicht Bereiche eingestanzt, ohne die Schutzschicht zu durchstanzen. Die Ausmaße der Bereiche entsprechen in etwa denen unterschiedlicher Fingernägel.
Die Zwischenstege zwischen den Bereichen werden abgegittert.

7. Das nach 6) erhaltene Laminat wird durch Schneiden derart zerteilt, daß man Laminat-Karten, nachfolgend als Karten bezeichnet, erhält, die mindestens 10 Bereiche aufweisen.

(je ein Bereich für einen Fingernagel von zwei Händen)

Das so hergestellte Laminat erwies sich bei einer Lagerung von 6 Monaten bei 50 °C als stabil und erfüllte die geforderten Applikations- und Trageeigenschaften. Produkte ohne Abdeckschicht werden nach Lagerung von 2 Tagen bei 50 °C spröde und lassen sich nicht mehr applizieren.
Die entsprechend dem obigen Beispiel hergestellten Laminate werden wie folgt auf den Nagel appliziert:
Zur Behandlung eines Nagels wird ein Bereich mittels der Abziehhilfe von der Schutzschicht abgelöst und auf dem Nagel aufgeklebt. Anschließend bzw. gleichzeitig mit dem Aufkleben wird die Abdeckschicht entfernt und dann die selbstklebde Polymerschicht auf dem Nagel angeformt.
Für den Fall, daß die Abdeckschicht mehrere Bereiche zusammenhängend überdeckt, wird die Abdeckschicht kurzzeitig entfernt, um die selbstklebende Polymerschicht von der Schutzschicht abzulösen.
Die selbstklebende Polymerschicht weist nach der Applikation die gleichen Trageeigenschaften auf wie die Lackschicht, aufgetragen durch die lösungsmittelhaltige Nagellackmasse, wobei im Gegensatz zum Aufkleben sogenannter künstlicher Fingernägel kein Fremdkörpergefühl auftritt.
Die selbstklebende Polymerschicht läßt sich von einem Nagel entfernen, indem man eine selbsthaftende Folie auf die Polymerschicht überstehend aufklebt und nach Andrücken der Folie an den Rändern der Polymerschicht diese insgesamt vom Nagel abzieht.

(Entfernung von Preisetiketten auf Verpackungen mittels eines Selbstklebebandes)

Auf dem Nagel verbleibt kein Kleberrest der selbstklebenden Polymerschicht, so daß eine Reinigung des Nagels mit einem organischen Lösungsmittel unnötig ist.
Durch dieses Applikationssystem (Laminat) wird ebenfalls die bei Nagellacken oft auftretende Gefahr des sogenannten "staining", Penetration von Farbstoff in den Nagel, verhindert.
Die Erfindung wird ferner anhand der nachfolgenden Figuren näher erläutert:
Figur 1 stellt eine Karte mit drei Bereichen im Querschnitt dar.
Figur 2 zeigt eine Abdeckschicht, die übergreifend mehrere Bereiche abdeckt.
Figur 3 zeigt die Aufsicht auf drei Bereiche die übergreifend mit der Abdeckschicht bedeckt sind.
Figur 4 zeigt die Rückseite der Schutzschicht mit durchstanzter Abziehhilfe.

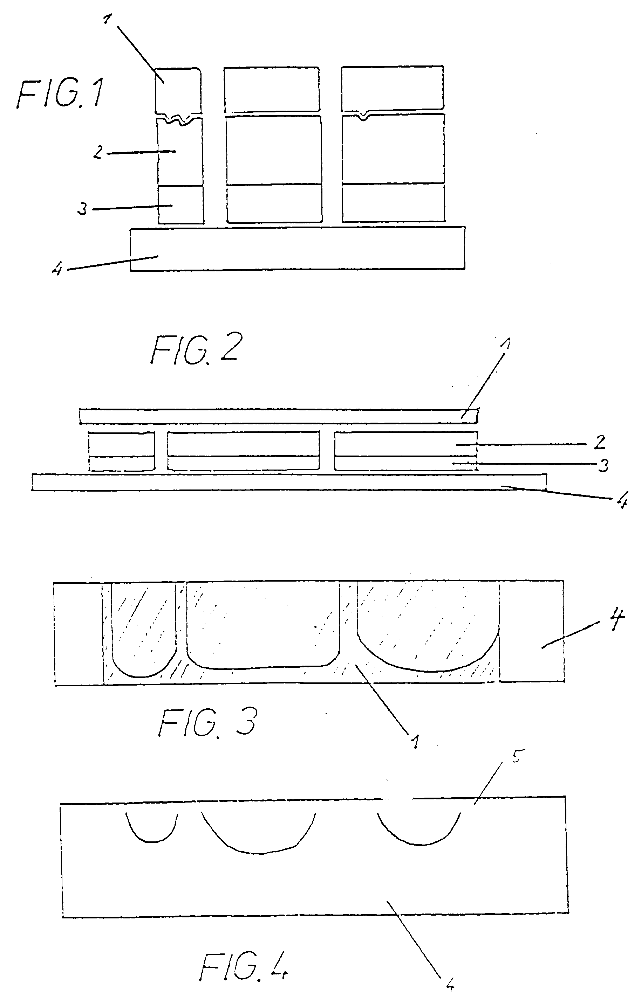

In Fig. 1 ist auf einer Abdeckschicht 1 (Polyesterfolie einer Dicke von 100 μm mit zum Teil strukturierter Oberfläche, die vor der Applikation zu entfernen ist), eine Polymerschicht 2 (Nagellack auf der Basis von Nitrocellulose, pigmentiert, Schichtdicke 200 μm und auf der Polymerschicht eine Kleberschicht 3 (auf der Basis von Polyisobutylen, Schichtdicke 80 μm angeordnet). Abgeschlossen wird das Laminat von einer Schutzschicht 4.
Fig. 2 zeigt eine Abdeckschicht 1-, die mehrere Bereiche abdeckt.
Fig. 3 zeigt die Aufsicht auf drei Bereiche, die übergreifend mit der Abdeckschicht bedeckt sind.
Fig. 4 zeigt die Rückseite der Schutzschicht 4 mit durchstanzter Abziehhilfe 5.

**Patentansprüche**

1.    Selbstklebendes, wenigstens einen Weichmacher enthaltendes, an Fuß- und/oder Fingernägel anformbares Laminat, enthaltend

    a) eine Farbstoffe und/oder Pigmente enthaltende filmbildende Polymerschicht

    b) eine darauf befindliche Haftkleberschicht und

    c) eine die Haftkleberschicht abdeckende, wiederablösbare, vorzugsweise silikonisierte Schutzfolie, dadurch gekennzeichnet, daß die filmbildende Polymerschicht auf der der Haftkleberschicht entgegengesetzten Seite mit einer abdeckenden, ablösbaren, gegenüber den übrigen Bestandteilen des Laminats und den bei der Herstellung des Laminats verwendeten Materialien resistenten Abdeckfolie versehen ist, wobei die vor Applikation zu entfernende Abdeckfolie auf einer beschichteten Seite adhäsiv ausgerüstet ist und die Adhäsion der Abdeckfolie an der Polymerschicht geringer ist, als die Adhäsion zwischen Kleberschicht und Schutzfolie.

2.    Selbstklebendes Laminat nach Anspruch 1, dadurch gekennzeichnet, daß die Abdeckfolie strukturiert ist.

3.    Selbstklebendes Laminat nach Anspruch 1 oder 2, dadurch gekennzeichnet, daß die Abdeckfolie aus Kunststoff besteht.

4.    Selbstklebendes Laminat nach einem der Ansprüche 1 bis 3, dadurch gekennzeichnet, daß die Abdeckfolie mit einer Abziehhilfe versehen ist.

5.    Selbstklebendes Laminat nach einem der Ansprüche 1 bis 4, dadurch gekennzeichnet, daß es als Weichmacher Citronensäureester enthält.

6.    Selbstklebendes Laminat nach einem der Ansprüche 1 bis 5, dadurch gekennzeichnet, daß es als Weichmacher Campher enthält.

7.    Selbstklebendes Laminat nach einem der Ansprüche 1 bis 6, dadurch gekennzeichnet, daß die Konzentration des Weichmachers oder der Weichmacher in der Polymerschicht 1 bis 30 Gew.-%, vorzugsweise 5 bis 12,5 Gew.-% und insbesondere 7 bis 10 Gew.-%, bezogen auf die Polymere der Schicht bzw. der Schichten, beträgt.

8.    Selbstklebendes Laminat nach einem der Ansprüche 1 bis 7, dadurch gekennzeichnet, daß die Polymerschicht und/oder die Kleberschicht eine geruchsaktive Komponente enthält.

9.    Selbstklebendes Laminat nach einem der Ansprüche 1 bis 8, dadurch gekennzeichnet, daß die Kleberschicht und/oder die Polymerschicht antimykotisch wirksame Stoffe enthält.

10.    Selbstklebendes Laminat nach Anspruch 9, dadurch gekennzeichnet, daß die Kleber- und/oder die Polymerschicht als antimykotische Wirkstoffe Clotrimazol, Miconazol, Ketoconazol, Econazol, Naftifin, Ciclopiroxolamin, Fenticlor, Sulbentin, Tolnaftat und/oder Haloprogin enthält.

11.    Selbstklebendes Laminat nach einem der Ansprüche 1 bis 10, dadurch gekennzeichnet, daß die Kleberschicht einen die Penetration des Wirkstoffes in den Nagel fördernden Anteil enthält, wie DMSO, DMF.

12.    Selbstklebendes Laminat nach einem der Ansprüche 1 bis 11, dadurch gekennzeichnet, daß das Flächengewicht des Klebers 20 bis 100 g/m$^2$, vorzugsweise 25 bis 75 g/m$^2$ beträgt.

13.    Selbstklebendes Laminat nach einem der Ansprüche 1 bis 12, dadurch gekennzeichnet, daß die Polymerschicht eine Schichtdicke von 25 bis 200 μm, vorzugsweise 60 bis 150 μm aufweist.

14.    Selbstklebendes Laminat nach einem der Ansprüche 1 bis 13, dadurch gekennzeichnet, daß die Polymerschicht aus Nitrozellulose und/oder Acrylaten bestehen oder Nitrozellulose und/oder Acrylate enthalten, wobei es sich bei den Acrylaten um Polymere handelt, bestehend aus Acrylsäure und/oder Dimethylaminoethylmethacrylat und/oder Methylacrylsäureestern.

15.    Selbstklebendes Laminat nach Anspruch 14, dadurch gekennzeichnet, daß die Polymerschichten unterschiedlich zusammengesetzt sind.

16.    Verfahren zur Herstellung eines selbstklebenden, an Fuß- oder Fingernägel anformbaren Laminats nach einem der Ansprüche 1 bis 15, dadurch gekennzeichnet, daß man einen Lack, der Farbstoffe und/oder Pigmente, wenigstens einen Weichmacher und wenigstens ein filmbildendes Polymer, gelöst in einem organischen Lösungsmittel enthält, auf eine entfernbare Abdeckfolie aufbringt, die wenigstens die gleiche Größe wie das Laminat hat, den Lack unter Rückgewinnung des Lösungsmittels trocknet und die mit dem getrockneten Lack beschichtete Abdeckfolie lackseitig mit einem Kleberfilm und einer Schutzschicht laminiert.

17. Verwendung des Laminats nach einem der Ansprüche 1 bis 15 zur Aufbringung auf Fuß- oder Fingernägel als künstliche Nägel.

**Claims**

1. A self-adhesive laminate containing at least one plasticizer and being formable to toenails and/or fingernails comprising
   a) a film-forming polymeric layer containing dyestuffs and/or pigments,
   b) a pressure-sensitive adhesive layer located thereon, and
   c) a removable, preferably siliconized protective film covering the pressure-sensitive adhesive layer, characterized in that the film-forming polymeric layer is covered on the face farther from the pressure-sensitive adhesive layer by a detachable covering film being resistant towards the other components of the laminate and towards the materials used in the production of the laminate, whereby the covering film, which is to be removed prior to application, is provided with an adhesive coating on a coated side, and whereby the adhesion of the covering film to the polymeric layer is less than the adhesion between the adhesive layer and the protective film.

2. The self-adhesive laminate according to claim 1, characterized in that the covering film has a structure.

3. The self-adhesive laminate according to claim 1 or 2, characterized in that the covering film consists of a plastics material.

4. The self-adhesive laminate according to any one of claims 1 to 3, characterized in that the covering film is provided with a peel-off aid.

5. The self-adhesive laminate according to any one of claims 1 to 4, characterized in that it contains citric ester as plasticizer.

6. The self-adhesive laminate according to any one of claims 1 to 5, characterized in that it contains camphor as plasticizing agent.

7. The self-adhesive laminate according to any one of claims 1 to 6, characterized in that the concentration of the plasticizer or plasticizers within the polymeric layer amounts to 1 to 30%-wt., preferably 5 to 12.5%-wt., and, in particular, 7 to 10%-wt., relative to the polymers of the layer or layers.

8. The self-adhesive laminate according to any one of claims 1 to 7, characterized in that the polymeric layer and/or the adhesive layer contain an odour-active component.

9. The self-adhesive laminate according to any one of claims 1 to 8, characterized in that the adhesive layer and/or the polymeric layer comprise antimycotically effective substances.

10. The self-adhesive laminate according to claim 9, characterized in that the adhesive and/or polymeric layer comprise as antimycotics active substances, such as clotrimazole, miconazole, ketoconazole, econazole, naftifin, ciclopiroxolamine, fenticlor, sulbentine, tolnaftate, and/or haloprogin.

11. The self-adhesive laminate according to any one of claims 1 to 10, characterized in that the adhesive layer comprises a component promoting the penetration of the active substance into the nail, such as DMSO or DMF.

12. The self-adhesive laminate according to any one of claims 1 to 11, characterized in that the weight per area of the adhesive amounts to 20 to 100 $g/m^2$, preferably 25 to 75 $g/m^2$.

13. The self-adhesive laminate according to any one of claims 1 to 12, characterized in that the polymeric layer has a thickness of 25 to 200 $\mu m$, preferably 60 to 150 $\mu m$.

**14.** The self-adhesive laminate according to any one of claims 1 to 13, characterized in that the polymeric layer(s) consist of nitrocellulose and/or acrylates, or contain nitrocellulose and/or acrylates, whereby the acrylates are polymers consisting of acrylic acid and/or dimethylaminoethyl methacrylate and/or methyl acrylic acids.

**15.** The self-adhesive laminate according to claim 14, characterized in that the polymeric layers are of different compositions.

**16.** A process for the production of a self-adhesive laminate which can be shaped to toenails or fingernails as defined in any one of claims 1 to 15, characterized in that a lacquer containing colorants and/or pigments, at least one plasticizer, and at least one film-forming polymer, dissolved in an organic solvent, is applied on a removable covering film having at least the same size as the laminate, the lacquer is dried under solvent recovery, and the lacquer-coated side of the covering film, which is coated with the dried lacquer, is laminated with an adhesive film and a protective layer.

**17.** Use of the laminate as defined in any one of claims 1 to 15 for applying it as artificial nails on toenails or fingernails.

**Revendications**

**1.** Lamifié autocollant, contenant au moins un plastifiant, adaptable en forme aux ongles des pieds et des doigts, qui contient
a) une couche polymérique, filmogène, contenant des colorants et/ou des pigments,
b) une couche autocollante ou collant par contact par-dessus la première couche et
c) une feuille de protection recouvrant la couche collant par contact ou autocollante, amovible, de préférence siliconée,
caractérisé en ce que la couche polymérique filmogène est pourvue sur le côté opposé à la couche autocollante ou collant par contact, d'une feuille de couverture recouvrante, amovible, résistant aux autres constituants du lamifié ainsi qu'aux matières utilisées pour la confection du stratifié, où la feuille de couverture à éliminer avant l'application est rendue adhésive sur un côté revêtu et en ce que l'adhérence de la feuille de couverture à la couche polymérique est plus faible que l'adhérence entre la couche de colle et la feuille de protection.

**2.** Lamifié autocollant suivant la revendication 1, caractérisé en ce que la feuille de couverture est structurée.

**3.** Lamifié autocollant suivant la revendication 1 ou 2, caractérisé en ce que la feuille de couverture est constituée d'une matière plastique.

**4.** Lamifié autocollant suivant l'une quelconque des revendications 1 à 3, caractérisé en ce que la feuille de couverture est pourvue d'un aide d'arrachement.

**5.** Lamifié autocollant suivant l'une quelconque des revendications 1 à 4, caractérisé en ce qu'il contient un ester de l'acide citrique à titre de plastifiant.

**6.** Lamifié autocollant suivant l'une quelconque des revnedications 1 à 5, caractérisé en ce qu'il contient du camphre à titre de plastifiant.

**7.** Lamifié autocollant suivant l'une quelconque des revendications 1 à 6, caractérisé en ce la concentration du plastifiant ou des plastifiants dans la couche polymérique varie de 1 à 30% en poids, de préférence 5 à 12,5% en poids et, plus particulièrement, 7 à 10% en poids, par rapport aux polymères de la couche ou des couches.

**8.** Lamifié autocollant suivant l'une quelconque des revendications 1 à 7, caractérisé en ce que la couche polymérique et/ou la couche collante contiennent un composant olfactif.

**9.** Lamifié autocollant suivant l'une quelconque des revendications 1 à 8, caractérisé en ce que la couche collante et/ou la couche polymérique contiennent des substances antimycotiques.

10. Lamifié autocollant suivant la revendication 9, caractérisé en ce que la couche collante et/ou la couche polymérique contiennent, à titre de substances antimycotiques, le clotrimazole, le miconazole, le cétoconazole, l'éconazole, la naphtifine, la cyclopiroxolamine, le fenticlor, la sulbentine, le tolnaphtate et/ou l'haloprogine.

11. Lamifié autocollant suivant l'une quelconque des revendications 1 à 10, caractérisé en ce que la couche collante contient une fraction favorisant la pénétration du principe actif dans l'ongle, tel que du DMSO ou du DMF.

12. Lamifié autocollant suivant l'une quelconque des revendications 1 à 11, caractérisé en ce que le grammage de la colle varie de 20 à 100 $g/m^2$, de préférence de 25 à 75 $g/m^2$.

13. Lamifié autocollant suivant l'une quelconque des revendications 1 à 12, caractérisé en ce que la couche polymérique possède une épaisseur de 25 à 200 μm, de préférence de 60 à 150 μm.

14. Lamifié autocollant suivant l'une quelconque des revendications 1 à 13, caractérisé en ce que la couche polymérique se compose de nitrocellulose et/ou d'acrylates, ou contient de la nitrocellulose et/ou des acrylates, où il s'agit, dans le cas des acrylates, de polymères qui sont constitués d'acide acrylique et/ou de méthacrylate de diméthyaminoéthyle et/ou d'esters de l'acide méthacrylique.

15. Lamifié autocollant suivant la revendication 14, caractérisé en ce que les couches polymériques sont de compositions différentes.

16. Procédé de préparation d'un lamifié autocollant, adaptable en forme aux ongles des pieds ou des doigts suivant l'une quelconque des revendications 1 à 15, caractérisé en ce que l'on applique un vernis qui contient des colorants et/ou des pigments, au moins un plastifiant et au moins un polymère filmogène, dissous dans un solvant organique, sur une feuille de couverture amovible, qui possède au moins la même taille que le lamifié, on sèche le vernis sous récupération du solvant et on lamifie la feuille de couverture revêtue de vernis séché côté vernis avec un film collant et une couche de protection.

17. Utilisation du lamifié suivant l'une quelconque des revendications 1 à 15 en vue de son application sur les ongles des pieds et des doigts à titre de faux ongles.

9

EP 0 542 786 B1

FIG.1

1
2
3
4

FIG.2

1
2
3
4

FIG.3

4
1

FIG.4

5
4

10